# EUROPEAN PATENT SPECIFICATION

(11) **EP 3 610 037 B1**
(45) Date of publication and mention of the grant of the patent: **06.10.2021**
(21) Application number: 18814162.6
(22) Date of filing: 28.03.2018
(51) Int. Cl.: C12Q 1/6806, C12Q 1/6825, B01L 3/00, B01L 7/00

(54) **PAPER-BASED ANALYTICAL MICROFLUIDIC CHIP AND DEVICE, HAVING EMBOSSED&DEBOSSED PROCESSING CHANNEL, FOR NUCLEIC ACID DIAGNOSIS**
PAPIERBASIERTER ANALYTISCHER MIKROFLUIDISCHER CHIP MIT GEPRÄGTEN UND EINGESTANZTEN VERARBEITUNGSKANÄLEN ZUR NUKLEINSÄUREDIAGNOSE
DISPOSITIF ET PUCE MICROFLUIDIQUES ANALYTIQUES SUR SUPPORT PAPIER, AYANT UN CANAL DE TRAITEMENT BOSSELÉ EN RELIEF ET EN CREUX, POUR LE DIAGNOSTIC D'ACIDES NUCLÉIQUES

(30) Priority: 12.04.2017 TR 201705423
(43) Date of publication of application: 19.02.2020
(73) Proprietor: Karakaya, Melike, Bornova/Izmir (TR)
(72) Inventor: KARAKAYA, Sait Fatih, Bornova/Izmir (TR); KARAKAYA, Melike, Bornova/Izmir (TR)
(74) Representative: Kaya, Erdem
(86) International application number: PCT/TR2018/050126
(87) International publication number: WO 2018/226193

(56) References cited:
- US-A1- 2014 335 527
- US-A1- 2016 327 510
- KOKKINOS CHRISTOS ET AL: "Emerging trends in biosensing using stripping voltammetric detection of metal-containing nanolabels - A review", ANALYTICA CHIMICA ACTA, ELSEVIER, AMSTERDAM, NL, vol. 961, 24 January 2017 (2017-01-24), pages 12-32, XP029927210, ISSN: 0003-2670, DOI: 10.1016/J.ACA.2017.01.016
- JARUWAN METTAKOONPITAK ET AL: "Electrochemistry on Paper-based Analytical Devices: A Review", ELECTROANALYSIS, vol. 28, no. 7, 1 July 2016 (2016-07-01), pages 1420-1436, XP055569055, US ISSN: 1040-0397, DOI: 10.1002/elan.201501143
- NATALIA M. RODRIGUEZ ET AL: "A fully integrated paperfluidic molecular diagnostic chip for the extraction, amplification, and detection of nucleic acids from clinical samples", LAB ON A CHIP, vol. 16, no. 4, 1 January 2016 (2016-01-01), pages 753-763, XP055567189, ISSN: 1473-0197, DOI: 10.1039/C5LC01392E
- XIANG LI ET AL: "Detection of Hepatitis B Virus DNA with a Paper Electrochemical Sensor", ANALYTICAL CHEMISTRY, vol. 87, no. 17, 10 August 2015 (2015-08-10), pages 9009-9015, XP055569046, US ISSN: 0003-2700, DOI: 10.1021/acs.analchem.5b02210
- JOHN T. CONNELLY ET AL: ""Paper Machine" for Molecular Diagnostics", ANALYTICAL CHEMISTRY, vol. 87, no. 15, 4 August 2015 (2015-08-04), pages 7595-7601, XP055330797, US ISSN: 0003-2700, DOI: 10.1021/acs.analchem.5b00411
- MELIKE KARAKAYA: "Analytical Molecular Diagnosis of Cervical Cancer via Paper Microfluidic Chip", PROCEEDINGS, vol. 2, no. 25, 20 December 2018 (2018-12-20), page 1556, XP055567129, DOI: 10.3390/proceedings2251556

## Description

### TECHNICAL FIELD

The present invention is related to develop a point of care device and chip that provides early diagnosis of cancer and infectious diseases. This invention uses nucleic acid biomarkers existing in blood, urine, tissue and similar clinical patient samples for the molecular diagnosis of cancer and infectious diseases in a short time and early manner.

### PRIOR ART

In the previous technologies of the molecular diagnosis of cancer and infectious diseases; sample preparation by binding nanoparticle and extraction of clinical sample take place in laboratory medium. There is a need for laboratory experiment extraction of 30 minutes and device centrifuge. Bioreagent binding process can take place via nanoparticle binding process in laboratory in long duration or via enzymatic immobilization in electrodes. Moreover, there are some enzyme-based paper microfluidic devices where extraction and sample preparation steps can be realized outside of paper chip. In these devices, paper base is used only in analytical diagnosis parts where fabrication and analysis parts are realized by similar techniques but in different methods.

In the patent study made in year 2013 and with number CN12980998A (1), a study is described where only the determination of biomarkers is realized by pastimmuno-sensors bonded to the paper surface. The invention is determination-focused and the electrode fabrication is realized by application of carbon-paste onto the paper surface via mold (stencil printed). As the reference electrode, silver chloride electrode is used instead of carbon electrode. In the device used in said invention, an enzymatic electrochemical measurement is provided, however the extraction of nucleic acid and preparation of sample do not take place in this device.

In the study made in year 2009 by Dungchai et al, with the title of "Electrochemical Detection for Paper-Based Microfluidics" and published in Analytical Chemistry with number 2009 Volume 81, No 14, July 15; and in the study made in year 2014 by Scida et al, with the title of "Simple, Sensitive, and Quantitative Electrochemical Detection Method for Paper Analytical Devices" and published in Analytical Chemistry with number 2014, 86, 6501-6507; and in the study made in year 2015 by Ling Yu and published in Royal Society of Chemistry with number Lab Chip, 2015, 15, 1642-1645; paper base is used, however, in production steps, pre-mold production is needed with lithography, photo-resist coating and UV-like needs. Moreover, these studies only comprise the diagnosis part, and the extraction and sample preparation (nanoparticle binding) processes which need laboratory equipment and experts in their field are also realized again in laboratory medium in long durations.

The invention with patent number US2016/327510 provides methods for the electrochemical detection of analytes.Methods for detecting an analyte can comprise flowing fluid along a channel to accumulate the analyte conjugated to a metal particle (i.e., an analyte conjugate) in a region of the channel in electrochemical contact with a working electrode. The channel can be, for example, a microfluidic channel. The analyte conjugate can be accumulated in the region of the channel in electrochemical contact with a working electrode by a localization element. The localization element can be any feature that is configured to increase the concentration of the analyte conjugate in the region of the channel in electrochemical contact with the working electrode in the presence of fluid flow through the channel.

Xiang Li Et Al: " Detection of Hepatit B Virus DANN with a Paper Electrochemical Sensor"; article describes that a simple paper-based electrochemical sensor, fabricated by paper folding, is able to detect a 30-base nucleotide sequence characteristic of DNA from the hepatitis B virus (HBV) with a detection limit of 85 pM. This device is based on design principles we have reported previously for detecting proteins via a metalloimmunoassay. It has four desirable attributes. First, its design combines simple origami (paper folding) assembly, the open structure of a hollow-channel paper analytical device to accommodate micrometer-scale particles, and a convenient slip layer for timing incubation steps. Second, two stages of amplification are achieved: silver nanoparticle labels provide a maximum amplification factor of 250 000 and magnetic microbeads, which are mobile solid-phase supports for the capture probes, are concentrated at a detection electrode and provide an additional ∼25-fold amplification. Third, there are no enzymes or antibodies used in the assay, thereby increasing its speed, stability, and robustness. Fourth, only a single sample incubation step is required before detection is initiated.

As a result, because of all of the abovementioned problems, an invention is required in the related technical field.

### BRIEF DESCRIPTION OF THE INVENTION

The present invention is defined in the appended claims.

The present invention relates to a diagnosis device as defined in the appended claims, which facilitates diagnosis of infectious diseases and clinical cancer screening, for eliminating the above mentioned disadvantages and for bringing new advantages to the related technical field.

The main object of the present invention is to provide a diagnosis device where the duration of cancer screening and infection diagnosis is shortened.

Another object of the present invention is to provide a diagnosis device with reduced cost.

Another object of the present invention is to provide a diagnosis device, which can make measurement in a single step.

Another object of the present invention is to provide a diagnosis device, which can make measurement without enzyme.

Another object of the present invention is to provide a diagnosis device, which does not need laboratory medium for application.

Another object of the present invention is to provide a diagnosis device where the extraction and analysis can be realized on a single chip and device.

Another object of the present invention is to provide a diagnosis device where the extraction process can be realized on the chip.

Another object of the present invention is to provide a diagnosis device where nucleic acid biomarkers can be applied.

Another object of the present invention is to provide a diagnosis device, which can realize measurement by using paper-based chip.

Another object of the present invention is to provide a diagnosis device, which can make measurement on the chip without the need for shearing and folding processes.

Another object of the present invention is to provide a diagnosis device, which can make voltametric measurement.

Another object of the present invention is to provide a diagnosis device where measurement duration is shortened.

In order to realize all of the abovementioned objects and the objects which come out from the detailed description below, disclosed is a diagnosis device developed for providing diagnosis of cancer and infectious diseases via nucleic acids existing in samples like blood, tissue, saliva, urine. Accordingly, said disclosed device is characterized by comprising:
- an extraction and hybridization channel where the nucleic acids, existing in the sample which is to be measured, are precipitated with glycoblue and where said nucleic acids are washed with washing liquids and where extraction and purification of nucleic acids are provided;
- a lock channel positioned in the vicinity of the extraction and hybridization channel and embodied such that liquid flow is provided and which provides removal of the liquids from here;
- a liquid absorption channel positioned in the vicinity of said lock channel and which absorbs the liquid existing in the lock channel;
- a chip comprising a diagnosis and determination channel which can realize measurement -by means of at least one electrode and positioned in the other vicinity of the extraction and hybridization channel;
- a chip housing wherein said chip is placed;
- a heat panel positioned in the vicinity of said chip housing and which provides heat to the chip;
- a slidable magnet positioned under said chip housing and which transfers the nucleic acids, which are nano-micro particle bonded, to said diagnosis and determination channel through the extraction and hybridization channel, and an electro-analytical measurement part having electrodes which can realize precise measurements at low limit of detection (LOD) and comprising graphene modified pencil lead..

According to the present invention, the chip is made of a material comprising cellulose.

Moreover, channels are integrated to each other and embodied as debossed and embossed onto the surface in a single step thanks to the usage of embossing printer. Thus, the paper chip comprising cellulose is produced in an easy, cheap and ergonomic manner.

In a preferred embodiment of the present invention, the extraction and hybridization channel, the lock channel and the diagnosis and determination channel are in debossed form and all of these processes can be realized on a single paper chip surface.

In another preferred embodiment of the present invention, the liquid absorption channel is in embossed form.

In another preferred embodiment of the present invention, the liquid absorption channel is in hydrophilic form.

In another preferred embodiment of the present invention, the extraction and hybridization channel, the lock channel and the diagnosis and determination channel are in hydrophobic form.

In another preferred embodiment of the present invention, the extraction of the nucleic acid from the sample is realized in the channel, provided on the paper chip, via single-step extraction-purification solution.

In another preferred embodiment of the present invention, the hybridization of the extracted nucleic acids with nanoparticle bonded primers is again provided inside the channel existing on the paper chip.

According to the present invention, the heat required for the hybridization process is provided by the measurement device wherein it is placed and nano-particle analyte is separated in the hybridization channel and this analyte is transferred to the diagnosis channel by means of the magnet apparatus existing in the device.

According to the present invention, there are graphene modified pencil lead electrodes in the diagnosis channel and these are placed into the paper channel.

In another preferred embodiment of the present invention, electrochemical diagnosis can be realized at low limit of detection (LOD).

According to the present invention, the measurement part comprises at least one display. Thus, the display can show the nucleic acid copy number in micro-liter and the load amounts of nanoparticles in the femto-molar level.

According to the present invention, the measurement part comprises an energy inlet and a keypad providing operation of the diagnosis device.

In order to realize all of the abovementioned objects and the objects which are to be deducted from the detailed description below, disclosed is a method developed for providing diagnosis of cancer and infectious diseases via nucleic acids in samples like blood, tissue, urine. Accordingly, said method is characterized by comprising the steps of:
(a) transferring the sample to an extraction and hybridization channel through an inlet opening embodied on a chip,
(b) extracting nucleic acids in said extraction and hybridization channel via guanidinium and alcohol including lysis and purification method and precipitating and washing said nucleic acids with glycoblue,
(c) flowing the liquids, existing in extraction and hybridization channel during step (b), to the lock channel and afterwards, beginning absorption by the liquid absorption channel,
(d) placing the chip to a chip housing,
(e) pushing the lock channel via mechanical effect and contacting the lock channel with an upper surface during step (d) in order to provide absorption of all liquid existing in the lock channel by the liquid absorption channel and at the same time, in order to provide interruption of the connection of the extraction and hybridization channel to the liquid absorption channel,
(f) transferring solution, comprising primers modified by bioreagent bonded metal and magnetic nano-micro particles, through the inlet opening onto the extracted nucleic acids precipitated into the extraction and hybridization channel,
(g) binding and hybridizing the extracted nucleic acids with the primers, modified by bioreagent bonded metal existing in the solution, and magnetic nano-micro particles by means of activating the heat panel and heating the extraction and hybridization channel,
(h) drawing the nano-micro particle bonded nucleic acids to the diagnosis and determination channel comprising oxidizing chemical buffer solution through the extraction and hybridization channel by means of the movement of the sliding magnet,
(i) electrochemically determining the hybridized nucleic acids inside the oxidizing solution with the electrodes as a result of applying voltage to the diagnosis and determination channel.

According to the present invention, extraction, washing, nano-particle binding and analytical diagnosis can be realized on a single paper chip surface connected to each other by means of channels,

In a preferred embodiment of the present invention, in step (i), the electrochemical determination is determined as nucleic acid copy number and as fM with the usage of software.

### BRIEF DESCRIPTION OF THE FIGURES

In Figure 1, a representative general view of the paper disposable chip is given.
In Figure 2, a representative view of the diagnosis device is given.

### REFERENCE NUMBERS

10 Diagnosis device
11 Chip
   111 Inlet opening
   112 Extraction and hybridization channel
   113 Lock channel
   114 Liquid absorption channel
   115 Diagnosis and determination channel
   116 Electrodes
   117 Upper surface
   118 Lower surface
12 Measurement part
   121 Chip housing
   122 Heat panel
   123 Slidable magnet
   124 Keypad
   125 Display
   126 Energy inlet

### DETAILED DESCRIPTION OF THE INVENTION

In this detailed description, the subject matter diagnosis device (10) is explained with references to examples without forming any restrictive effect only in order to make the subject more understandable.

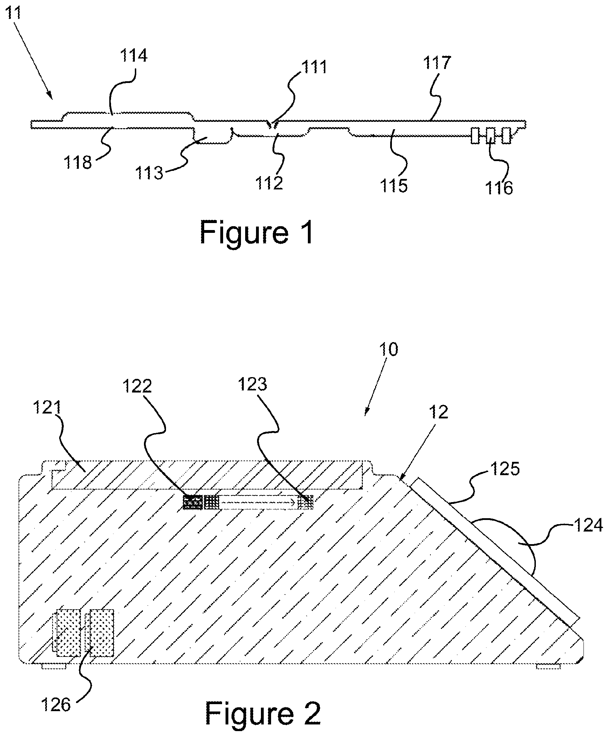

With reference to Figure 1 and 2, said diagnosis device (10) comprises a disposable chip (11) and a measurement part (12) wherein said chip (11) is placed and where measurement is realized. Said chip (11) has a cellulose-based structure. The chip (11) is preferably made of paper. The chip (11) comprises an inlet opening (111), an extraction and hybridization channel (112) positioned under said inlet opening (111), a lock channel (113) positioned in the vicinity of said extraction and hybridization channel (112), a diagnosis and determination channel (115) positioned in the other vicinity of the extraction and hybridization channel (112), a liquid absorption channel (114) positioned in the vicinity of the lock channel (113). There is at least one electrode (116) positioned at the continuation of the diagnosis and determination channel (115) and preferably positioned at the end part thereof. There is an upper surface (117) which at least partially covers the extraction and hybridization channel (112), the lock channel (113) and the diagnosis and determination channel (115). On the base of said liquid absorption channel (114), a lower surface (118) is formed which is positioned parallel or essentially parallel to said upper surface (117). The channels, which are in the vicinity of each other, are connected to each other. The channels, which are in the vicinity of each other, are embodied in a manner providing correlated and arranged flow with each other where the liquids provided therein can pass to each other. The chip (11) has at least partially a closed geometry except the inlet opening (111). The chip (11) is placed into the transparent coating surface with self-adhesion in order to avoid any sterilization process in the diagnosis device (10).

The extraction and hybridization channel (112), the lock channel (113) and the diagnosis and determination channel (115), provided on the chip (11), are embodied in debossed form. The extraction and hybridization channel (112), the lock channel (113) and the diagnosis and determination channel (115), provided on the chip (11), have hydrophobic structure. The depth of the extraction and hybridization channel (112) is smaller than the depth of the lock channel (113). Thus, the liquids easily flow into the lock channel (113), which is deeper than the extraction, and hybridization channel (112).

The liquid absorption channel (114) provided on the chip (11) is embodied in embossed form. The liquid absorption channel (114) provided on the chip (11) has hydrophilic structure. The debossed and embossed form on the chip (11) is provided by means of the embossing printer with the desired geometry and/or patterns. Thus, by means of the debossed and embossed form, channel-forming process is provided without needing any physical process like shearing, folding and adhering for the three-dimensional structure which provides liquid flow. The hydrophilic and hydrophobic surfaces are provided with wax printer. The chip (11) can be produced with the corresponding colors with the desired optional surface characteristics.

The measurement part (12) is in the form of a frame with thickness of approximately 5 cm and with length of 15 cm and it comprises a chip housing (121) embodied such that the chip (11) is placed therein, a heat panel (122) positioned in the vicinity of said chip housing (121), a slidable magnet (123) positioned in the vicinity of said heat panel (122) and under the chip housing (121), a keypad (124) where the start and stop commands are given to the system, a screen (125) where the measurement and analysis results can be monitored and an energy inlet (126) which provides energy input for the operation of the diagnosis device (10).

For measurement during application, the sample is flowed to the extraction and hybridization channel (112) through the inlet opening (111). The sample can be blood, tissue, urine, etc. The sample, taken to the extraction and hybridization channel (112), is precipitated with glycoblue which is a known method in the art, and afterwards, the sample is washed with washing liquids and the extraction and purification of nucleic acids are provided. By means of this single step system developed based on lysis and purification method including alcohol and guanidinium known in the art, the nucleic acids are extracted and precipitated with glycoblue and thereby, the nucleic acids can be captured in the extraction and hybridization channel (112). During washing, the used liquids flow from the extraction and hybridization channel (112) to the lock channel (113) and said liquids are absorbed by the hydrophilic liquid absorption channel (114) existing in the vicinity of the lock channel (113).

After the nucleic acids are extracted, the chip (11) is placed to the chip housing (121) existing in the measurement part (12). Meanwhile, the lock channel (113) is pushed by means of mechanical effect and it is contacted with the upper surface (117). Thus, all liquid existing in the lock channel (113) is absorbed by the liquid absorption channel (114) and at the same time, the connection between the extraction and hybridization channel (112) and the liquid absorption channel (114) is interrupted. By means of this, the back flow of the liquid is prevented and the extracted nucleic acids are captured inside the extraction and hybridization channel (112).

Solution comprising primers modified with metal and magnetic nano-micro particles where bioreagent is bonded through the inlet opening (111) is poured onto the extracted nucleic acids precipitated inside the extraction and hybridization channel (112). As the solution is poured onto the nucleic acids, the diagnosis device (10) is turned on and the heat panel (122) is activated and the extraction and hybridization channel (112) is heated. By means of the heating process, the extracted nucleic acids are bonded to each other by means of primers modified with bioreagent bonded metal and magnetic nano-micro particles existing inside the solution and thus, hybridization takes place. Hybridization takes place approximately in 10 minutes. Hybridization takes place in the extraction and hybridization channel (112) without enzyme. By means of this binding, which takes place completely without enzyme, a small amount of nucleic acid sample leads to pre-densifying via nano-micro particles.

During preparation of nano-micro particle primer solution, primers with bioreagent nano-micro particles and with compliant bioreagent with key lock models have been used. Thiol bioreagent primers combine with citrate coated nano-micro particles and form the target nano-micro particle primer and the required primer solution with nano-micro particles have been produced via binding streptavidin bonded nano-micro particles to the biotin bonded primers. The selected primers have been prepared in the suitable nucleotide arrangement, which can be bonded, to the two ends in the single chain of the target nucleic acid. Nano-micro particle bonded primers with metal and magnetic properties can be presented together with the chip (11) in a ready form.

Nucleic acids, prepared on the chip (11) and which are nano-micro particle bonded after hybridization, are drawn from the extraction and hybridization channel (112) to the diagnosis and determination channel (115) by means of the movement of the slidable magnet (123). By means of the electrodes (116) provided in the diagnosis and determination channel (115), cancer or infection diagnosis is provided via nucleic acid. In the subject matter diagnosis device (10), by using graphene modified pencil lead electrodes (116), which can realize precise measurements in low limit of detection (LOD), the need for a mold is eliminated and thus, less expensive, more rapid and easy production can be realized.

In the diagnosis and determination channel (115), oxidizing chemical buffer solution exists. Voltage is applied to the diagnosis and determination channel (115) as the diagnosis device (10) continues operation. As a result of the applied voltage, electrochemical diagnosis is realized which is based on the measurement of current and load values resulting from electron exchange which occurs between electrodes (116) and the magnetic and metal nano-micro particle bonded nucleic acids. In other words, the diagnosis device (10) applies voltage to the diagnosis and determination channel (115) by means of electrodes (116) and it makes measurement with electrochemical quantitative analysis based on current and load measurement. The resulting current can be displayed on the screen with using software as nucleic acid replicate number existing in µL and femto-molar (hereafter, it will be called as fM) level. In the diagnosis device (10), the required replicate number in microliter of the nucleic acids in the applied voltage amount and fM level of the bonded nano-micro particles have been defined beforehand via suitable software. The condition of whether the required replicate number in microliter of the nucleic acids in the applied voltage amount and fM level of the bonded nano-micro particles are under or above the defined values can also be shown on the display (125).

The subject matter diagnosis device (10) provides on-chip extraction by using very small amount of patient sample and hybridization of the obtained nucleic acid by means of nano-particles on-chip in the form of an electro-analytical analysis without enzyme thanks to the disposable chip (11) and kit, and the usage and fabrication of the subject matter diagnosis device (10) are easy, low-cost, precise, portative and it can realize quantitative amount analysis of nucleic acids in a short time with high precision.

The most important characteristic which distinguishes the invention from the prior paper-based electro-analytic micro-fluids is that the extraction of the clinic sample and the hybridization of the nucleic acid obtained as a result of extraction by means of bioreagent bonded nano-micro particle primers and all of the electrochemical determination processes of this nano-particle bonded sample without enzyme can be realized on the same paper-based device. Thanks to these characteristics, these three processes can be realized in a very short time (approximately 30 minutes) in laboratory without needing different devices and materials.

In alternative applications of the present invention, the result is sent to cellular telephone by means of the patient monitoring system. In another application, moreover, colormatic enzyme including paper chip (11) is added to the diagnosis part or it is impregnated and it is provided in a colorimetric process instead of electro-analytic process. Different conductive, magnetic fluorescence and similar nano-particles can be bonded to the sample or they can be used by means ofbinding to the paper chip (11) surface. In order to improve the suctioning power and hydrophilic, hydrophobic characteristics of the paper chip (11), new paper bases can be formed by means of nano-cellulosic materials. Different electrode (116) systems can be used. In the diagnosis device (10), changes can be made in the channel system providing bonded flow of the three processes and in the inter-channel transfers with the key lock model. In the diagnosis device (10), software is used which is suitable to the sample, nano-particle or analysis output. The bioreagents used in production of nano-particle bonded primer solution can be changed.

By means of the subject matter method and the subject matter diagnosis device (10), single step on-chip (11) extraction can be realized, and sample can be prepared without enzyme by using primers, which are nanoparticle bonded beforehand by means of hybridization.

## Claims

1. A diagnosis device (10) for providing diagnosis of cancer and infectious diseases by means of nucleic acids biomarkers in samples like blood, tissue, saliva, urine, wherein said diagnostic device includes the following elements that perform the extraction, sample preparation and diagnosis of cancer and infectious diseases with nucleic acids;
a) a chip (11) comprising:
- an extraction and hybridization channel, (112) where the nucleic acids existing in the sample which is to be measured are precipitated with glycoblue and where said nucleic acids, are washed by means of washing liquids and where extraction and purification of nucleic acids are provided;
- a lock channel (113) positioned in the vicinity of the extraction and hybridization channel (112) and embodied such that liquid flow is provided and which provides removal of the liquids from channel (112);
- a liquid absorption channel (114) positioned in the vicinity of said lock channel (113) and which absorbs the liquid in the lock channel (113);
- a diagnosis and determination channel (115) and which realizes measurement by means of graphene modified pencil lead electrodes (116) where the extraction and hybridization channel (112) is positioned in the other vicinity;
wherein the chip (11) is made of material comprising cellulose;
b) a measurement part (12) in which the chip (11) is inserted and contains the elements that perform the necessary measurement operations;
said measurement part (12) comprises a chip housing (121) embodied such that the chip (11) is placed therein, a heat panel (122) positioned in the vicinity of said chip housing (121) and which provides heat to the chip (11), a slidable magnet (123) positioned in the vicinity of said heat panel (122) and under the chip housing (121), a keypad (124) where the start and stop commands are given to the system, a screen (125) where the measurement and analysis results can be monitored and an energy inlet (126) which provides energy input for the operation of the diagnosis device (10); wherein the liquid absorption channel, the extraction and hybridization channel (112), the lock channel (114) and the diagnosis and determination channel (115) arc channels integrated to each other and embodied in a three-dimensional from onto surface in a single step thanks to the usage of embossing printer.

2. A diagnosis device (10) according to claim 1, **wherein** the extraction and hybridization channel (112), the lock channel (113) and the diagnosis and determination channel (115) are in debossed form.

3. A diagnosis device (10) according to claim 1, **wherein** the liquid absorption channel (114) is in embossed form.

4. A diagnosis device (10) according to claim 1, **wherein** the liquid absorption channel (114) is in hydrophilic form.

5. A diagnosis device (10) according to claim 1, **wherein** the extraction and hybridization channel (112), the lock channel (113) and the diagnosis and determination channel (115) are in hydrophobic form.

6. A diagnosis device (10) according to claim 1, **wherein** the measurement part (12) comprises at least one display (125).

7. A diagnosis device (10) according to claim 1, **wherein** the measurement part (12) comprises a keypad (124) providing operation of the diagnosis device (10).

8. A method for providing diagnosis of cancer and infectious diseases by means of nucleic acids in samples like blood, tissue, urine, **characterized by** comprising the steps of;
(a) transferring the sample to an extraction and hybridization channel (112) through an inlet opening (111) embodied on a chip (11),
(b) extracting nucleic acids in said extraction and hybridization channel (112) by means of guanidinium and alcohol including lysis and purification method and precipitating and washing with glycoblue,
(c) during step (b), the nucleic acids contained in the extraction and hybridization channel (112) remain in the extraction and hybridization channel (112), while the undetected fluids flow into the lock channel (113) and then begin to be absorbed by the liquid suction channel (114) and
placing the chip (11) to a chip housing (121),
(d) pushing the lock channel (113) by means of mechanical effect and contacting the lock channel (113) with an upper surface (117) during step (d) in order to provide absorption of all liquid existing in the lock channel (113) by the liquid absorption channel (114) and at the same time, in order to provide interruption of the connection of the extraction and hybridization channel (112) to the liquid absorption channel (114),
(e) transferring solution, comprising primers modified by bioreagent bonded metal and magnetic nano-micro particles, through the inlet opening (111) onto the extracted nucleic acids precipitated: into the extraction and hybridization channel (112),
(f) binding and hybridizing the extracted nucleic acids with the primers, modified by bioreagent bonded metal existing in the solution, and magnetic nano-micro particles by means of activating the heat panel (122) comprised in the chip housing (121) and via heating the extraction and hybridization channel (112),
(g) transferring the nano-micro particle bonded nucleic acids to the diagnosis and determination channel (115) comprising oxidizing chemical buffer solution through the extraction and hybridization channel (112) by means of the movement of the sliding magnet (123) comprised in the chip housing (121),
(h) electrochemically determining the hybridized nucleic acids inside the oxidizing solution by means of graphene modified pencil lead electrodes (116) as a result of applying voltage to the diagnosis and determination channel (115),
- wherein extraction, washing, nanoparticle binding and analytical diagnosis are realized on a single paper chip (11) surface connected to each other by means of channels.

9. A diagnosis method according to claim 8, **wherein** in step (h), the electrochemical determination is determined as nucleic acid copy number and as fM by means of usage of software.

## Patentansprüche

1. Eine Diagnosevorrichtung (10) zum Bereitstellen einer Diagnose von Krebs und Infektionskrankheiten mittels Nukleinsäure-Biomarkern in Proben wie Blut, Gewebe, Speichel, Urin, wobei die genannte Diagnosevorrichtung die folgenden Elemente umfasst, die die Extraktion, Probenvorbereitung und Diagnose von Krebs und Infektionskrankheiten mit Nukleinsäuren durchführen;
a) ein Chip (11) umfassend:
- einen Extraktions- und Hybridisierungskanal (112), in dem die in der zu messenden Probe vorhandenen Nukleinsäuren mit Glycoblau präzipitiert und die genannte Nukleinsäuren mittels Waschflüssigkeiten gewaschen werden und in dem eine Extraktion und Reinigung von Nukleinsäuren vorgesehen ist;
- einen Schleusenkanal (113), der in der Nähe des Extraktions- und Hybridisierungskanals (112) positioniert und derart ausgelegt ist, dass ein Flüssigkeitsstrom gewährleistet wird und der für die Entfernung der Flüssigkeiten aus dem Kanal (112) sorgt;
- einen Flüssigkeitsabsorptionskanal (114), der in der Nähe des Schleusenkanals (113) positioniert ist und der die Flüssigkeit in dem Schleusenkanal (113) absorbiert;
- einen Diagnose- und Bestimmungskanal (115), der eine Messung mittels graphenmodifizierter Bleistiftminenelektroden (116) realisiert; wobei der Extraktions- und Hybridisierungskanal (112) in der anderen Umgebung positioniert ist;
wobei der Chip (11) aus einem Material umfassend Zellulose besteht;
b) ein Messteil (12),
in den der Chip (11) eingesetzt ist und die Elemente, die die notwendigen Messvorgänge durchführen, umfasst;
der henannte Messteil (12) umfasst
ein Chipgehäuse (121), das derart ausgebildet ist, dass der Chip (11) darin platziert wird, eine Heizplatte (122), die in der Nähe des Chipgehäuses (121) positioniert ist und dem Chip (11) Wärme zuführt, einen verschiebbaren Magneten (123), der in der Nähe der Heizplatte (122) und unter dem Chipgehäuse (121) positioniert ist, eine Tastatur (124), wo die Start- und Stoppbefehle an das System gegeben werden, einen Bildschirm (125), auf dem die Mess- und Analyseergebnisse überwacht werden können und einen Energieeingang (126), der eine Energiezufuhr für den Betrieb der Diagnosevorrichtung (10) gewährleistet;
wobei der Flüssigkeitsabsorptionskanal, der Extraktions- und Hybridisierungskanal (112), der Schleusenkanal (114) und der Diagnose- und Bestimmungskanal (115) Kanäle sind, die ineinander integriert und und dreidimensional von der Oberfläche in einem einzigen Schritt durch den Einsatz eines Prägedruckers ausgebildet sind.

2. Eine Diagnosevorrichtung (10) nach Anspruch 1, **wobei** der Extraktions- und Hybridisierungskanal (112), der Schleusenkanal (113) und der Diagnose- und Bestimmungskanal (115) geprägt sind.

3. Eine Diagnosevorrichtung (10) nach Anspruch 1, **wobei** der Flüssigkeitsabsorptionskanal (114) in geprägter Form vorliegt.

4. Eine Diagnosevorrichtung (10) nach Anspruch 1, **wobei** der Flüssigkeitsabsorptionskanal (114) in hydrophiler Form vorliegt.

5. Eine Diagnosevorrichtung (10) nach Anspruch 1, **wobei** der Extraktions- und Hybridisierungskanal (112), der Schleusenkanal (113) und der Diagnose- und Bestimmungskanal (115) in hydrophober Form vorliegen.

6. Eine Diagnosevorrichtung (10) nach Anspruch 1, **wobei** der Messteil (12) mindestens eine Anzeige (125) umfasst.

7. Eine Diagnosevorrichtung (10) nach Anspruch 1, **wobei** der Messteil (12) eine Tastatur (124) umfasst, die den Betrieb der Diagnosevorrichtung (10) sicherstellt.

8. Ein Verfahren zur Bereitstellung der Diagnose von Krebs und Infektionskrankheiten mittels Nukleinsäuren in Proben wie Blut, Gewebe, Urin, **dadurch gekennzeichnet, dass** es die Schritte umfasst:
(a) Überführen der Probe zu einem Extraktions- und Hybridisierungskanal (112) über eine auf einem Chip (11) ausgebildete Einlassöffnung (111),
(b) Extraktieren von Nukleinsäuren in dem genanten Extraktions- und Hybridisierungskanal (112) mittels Guanidinium und Alkohol, einschließlich Lyse- und Reinigungsverfahren und Abscheiden und Waschen mit Glycoblau,
(c) während Schritt (b) verbleiben die im Extraktions- und Hybridisierungskanal (112) enthaltenen Nukleinsäuren im Extraktions- und Hybridisierungskanal (112), während die nicht nachgewiesenen Flüssigkeiten in den Schleusenkanal (113) fließen, und dann beginnen sie durch den Flüssigkeitssaugkanal (114) absorbiert zu werden und den Chip (11) in einem Chipgehäuse (121) zu platzieren,
(d) Schieben des Schleusenkanals (113) mittels mechanischer Wirkung und Kontaktieren des Schleusenkanals (113) mit einer oberen Fläche (117) während Schritt (d), um eine Absorption aller im Schleusenkanal (113) vorhandenen Flüssigkeit durch den Flüssigkeitsabsorptionskanal (114) zu sorgen und gleichzeitig um eine Unterbrechung der Verbindung des Extraktions- und Hybridisierungskanals (112) mit der Flüssigkeitsabsorptionskanal (114) zu sorgen,
(e) Übertragen einer Lösung, die einen Primer umfasst, die durch an Bioreagenzien gebundenen metalen und magnetischeb Nano-Mikropartikeln modifiziert sind, durch die Einlassöffnung (111) auf die in den Extraktions- und Hybridisierungskanal (112) ausgefällten extrahierten Nukleinsäuren,
(f) Binden und Hybridisieren der extrahierten Nukleinsäuren mit den Primern, die durch das in der Lösung vorhandenes bioreagenz-gebundenes Metall und den magnetischen Nano-Mikropartikeln mittels Aktivierung der im Chipgehäuse (121) enthaltenen Heizplatte (122) und durch Erhitzen des Extraktions- und Hybridisierungskanasl (112) modifiziert sind,
(g) Übertragen der an Nano-Mikropartikel gebundenen Nukleinsäuren in den Diagnose- und Bestimmungskanal (115), der eine oxidierende chemische Pufferlösung umfasst, durch den Extraktions- und Hybridisierungskanal (112) mittels der Bewegung des im Chipgehäuse (121) enthaltenen Gleitmagneten (123),
(h) elektro-chemisches Bestimmen der hybridisierten Nukleinsäuren innerhalb der Oxidationslösung mittels graphenmodifizierter Bleistiftminenelektroden (116) durch Anlegen einer Spannung an den Diagnose- und Bestimmungskanal (115),
- wobei die Extraktion, das Waschen, die Nanopartikel-Bindung und die analytische Diagnose auf einer einzigen, durch Kanäle miteinander verbundenen Oberfläche des Papierchips (11) realisiert werden können.

9. Ein Diagnoseverfahren nach Anspruch 8, **wobei** in Schritt (h) die elektro-chemische Bestimmung als Nukleinsäure-Kopienzahl und als fM mittels Software bestimmt wird.

## Revendications

1. Dispositif de diagnostic (10) pour fournir un diagnostic du cancer et des maladies infectieuses au moyen de biomarqueurs d'acides nucléiques dans des échantillons tels que le sang, les tissus, la salive, l'urine, dans lequel ledit dispositif de diagnostic comprend les éléments suivants qui effectuent l'extraction, la préparation de l'échantillon et le diagnostic du cancer et des maladies infectieuses avec des acides nucléiques ;
a) une puce (11) comprenant :
- un canal d'extraction et d'hybridation (112) où les acides nucléiques existant dans l'échantillon qui doit être mesuré sont précipités avec du glycoblue et où lesdits acides nucléiques sont lavés au moyen de liquides de lavage et où l'extraction et la purification des acides nucléiques sont prévues ;
- un canal de verrouillage (113) positionné à proximité du canal d'extraction et d'hybridation (112) et conçu de telle sorte qu'un écoulement de liquide est fourni et qui assure le retrait des liquides du canal (112) ;
- un canal d'absorption de liquide (114) positionné à proximité dudit canal de verrouillage (113) et qui absorbe le liquide dans le canal de verrouillage (113) ;
- un canal de diagnostic et de détermination (115) et qui réalise une mesure au moyen d'électrodes de mine de crayon modifiées par graphène (116) où le canal d'extraction et d'hybridation (112) est positionné dans l'autre proximité ;
dans lequel la puce (11) est faite d'un matériau comprenant de la cellulose ;
b) une partie de mesure (12) dans laquelle la puce (11) est insérée et contient les éléments qui effectuent les opérations de mesure nécessaires ;
ladite partie de mesure (12) comprend un boîtier de puce (121) conçu de telle sorte que la puce (11) est placée à l'intérieur, un panneau thermique (122) positionné à proximité dudit boîtier de puce (121) et qui fournit de la chaleur à la puce (11), un aimant coulissant (123) positionné à proximité dudit panneau thermique (122) et sous le boîtier de puce (121), un clavier (124) où les commandes de démarrage et d'arrêt sont données au système, un écran (125) où les résultats de mesure et d'analyse peuvent être surveillés et une entrée d'énergie (126) qui fournit une entrée d'énergie pour le fonctionnement du dispositif de diagnostic (10) ;
dans lequel le canal d'absorption de liquide, le canal d'extraction et d'hybridation (112), le canal de verrouillage (114) et le canal de diagnostic et de détermination (115) sont des canaux intégrés les uns aux autres et conçus sous une forme tridimensionnelle sur la surface en une seule étape grâce à l'utilisation d'une imprimante à gaufrage.

2. Dispositif de diagnostic (10) selon la revendication 1, **dans lequel** le canal d'extraction et d'hybridation (112), le canal de verrouillage (113) et le canal de diagnostic et de détermination (115) sont sous forme gravée.

3. Dispositif de diagnostic (10) selon la revendication 1, **dans lequel** le canal d'absorption de liquide (114) est sous forme gaufrée.

4. Dispositif de diagnostic (10) selon la revendication 1, **dans lequel** le canal d'absorption de liquide (114) est sous forme hydrophile.

5. Dispositif de diagnostic (10) selon la revendication 1, **dans lequel** le canal d'extraction et d'hybridation (112), le canal de verrouillage (113) et le canal de diagnostic et de détermination (115) sont sous forme hydrophobe.

6. Dispositif de diagnostic (10) selon la revendication 1, **dans lequel** la partie de mesure (12) comprend au moins un écran (125).

7. Dispositif de diagnostic (10) selon la revendication 1, **dans lequel** la partie de mesure (12) comprend un clavier (124) assurant le fonctionnement du dispositif de diagnostic (10).

8. Procédé pour fournir un diagnostic du cancer et des maladies infectieuses au moyen de biomarqueurs d'acides nucléiques dans des échantillons tels que le sang, les tissus, la salive, l'urine, **caractérisé en ce qu'**il comprend les étapes suivantes:
(a) transférer l'échantillon vers un canal d'extraction et d'hybridation (112) à travers une ouverture d'entrée (111) incorporée sur une puce (11),
(b) l'extraction des acides nucléiques dans ledit canal (112) d'extraction et d'hybridation au moyen de guanidinium et d'alcool comprenant un procédé de lyse et de purification et la précipitation et le lavage avec du glycoblue,
(c) pendant l'étape (b), les acides nucléiques contenus dans le canal d'extraction et d'hybridation (112) restent dans le canal d'extraction et d'hybridation (112) tandis que les fluides non détectés s'écoulent dans le canal de verrouillage (113) et commencent ensuite à être absorbés par le canal d'aspiration de liquide (114) et à placer la puce (11) dans un boîtier de puce (121),
(d) pousser le canal de verrouillage (113) au moyen d'un effet mécanique et mettre en contact le canal de verrouillage (113) avec une surface supérieure (117) pendant l'étape (d) afin d'assurer l'absorption de tout le liquide existant dans le canal de verrouillage (113) par le canal d'absorption de liquide (114) et en même temps, afin d'assurer l'interruption de la connexion du canal d'extraction et d'hybridation (112) au canal d'absorption de liquide (114),
(e) transférer la solution, comprenant des amorces modifiées par des nanoparticules métalliques et magnétiques liées par un bioréactif, à travers l'ouverture d'entrée (111) sur les acides nucléiques extraits précipités dans le canal d'extraction et d'hybridation (112),
(f) lier et hybrider les acides nucléiques extraits avec les amorces, modifiées par le métal lié par un bioréactrf existant dans la solution, et les nanoparticules magnétiques au moyen de l'activation du panneau thermique (122) compris dans le boîtier de la puce (121) et via le chauffage du canal d'extraction et d'hybridation (112),
(g) transférer les acides nucléiques liés par des nanoparticules vers le canal de diagnostic et de détermination (115) comprenant une solution tampon chimique oxydante à travers le canal d'extraction et d'hybridation (112) au moyen du mouvement de l'aimant coulissant (123) compris dans le boîtier de puce (121),
(h) déterminer électrochimiquement les acides nucléiques hybridés à l'intérieur de la solution oxydante au moyen d'électrodes de mine de crayon modifiées par graphène (116) suite à l'application d'une tension au canal de diagnostic et de détermination (115),
- dans lequel l'extraction, le lavage, la liaison des nanoparticules et le diagnostic analytique sont réalisés sur une seule surface de puce en papier (11) reliée les unes aux autres par des canaux.

9. Procédé de diagnostic selon la revendication 8, **dans lequel** à l'étape (h), la détermination électrochimique est déterminée en tant que nombre de copies d'acide nucléique et en tant que fM au moyen de l'utilisation d'un logiciel.
